# EUROPEAN PATENT APPLICATION

(11) **EP 4 560 025 A1**
(43) Date of publication of application: **28.05.2025**
(21) Application number: 23843088.8
(22) Date of filing: 21.07.2023
(51) Int. Cl.: C12Q 1/04, C12Q 1/48, C12Q 1/66, C12N 9/02, C12N 9/12

(54) **METHOD AND KIT FOR DETECTING MICROORGANISM OR CELL, OR DETECTING MICROORGANISM RELATED SUBSTANCE OR CELL RELATED SUBSTANCE**

(30) Priority: 22.07.2022 JP 2022116858
(71) Applicant: Kikkoman Corporation, Noda-shi, Chiba 278-8601 (JP)
(72) Inventor: ICHIYANAGI Yuko, Noda-shi, Chiba 278-8601 (JP); SUZUKI Shigeya, Noda-shi, Chiba 278-8601 (JP)
(74) Representative: J A Kemp LLP
(86) International application number: PCT/JP2023/026836
(87) International publication number: WO 2024/019165

(57) **Abstract**

An object is to provide a simple method and kit for detecting a microorganism or cell. A method for detecting a microorganism or cell, including the steps of first extracting the microorganism or cell with an extracting agent containing a surfactant; then collecting a sample with a sampling portion containing a surfactant adsorbent; and measuring luminescence level with a luminescence reagent, and a kit for the method are provided.

## Description

### Technical Field

The present invention relates to a method and a kit for detecting a microorganism or cell, or detecting a microorganism-related substance or a cell-related substance.

### Background Art

Adenosine triphosphate (hereinafter, referred to as ATP) is a nucleotide found in all living bodies, and is used by cells as a substrate that stores and releases energy. Microorganism and cells can be detected by measuring ATP.

Known representative methods for measuring ATP include a method comprising allowing ATP and the substrate, luciferin to react in the presence of luciferase, and measuring luminescence (Non Patent Literature 1). This reaction is catalyzed by luciferase and occurs in the presence of a divalent metal ion as follows.

[Formula] Luciferin + ATP + O₂ → oxyluciferin + adenosine monophosphate (AMP) + pyrophosphoric acid (PPi) + CO₂ + light

Luciferase is found in bacteria, protozoans, mollusks, insects, and the like. Examples of the insects having luciferase include beetles, for example, fireflies and click beetles. Numerous luciferase genes have been isolated and their nucleotide sequences have been also determined.

A conventional method for measuring cell-derived ATP comprises a step of performing sampling, and a step of performing extraction of a sampled sample. The sampling is a step of collecting a microorganism (and thus ATP contained in the microorganism). The extraction step is a step in which ATP within a microorganism contained in the collected sample is released extracellularly so that the ATP can be measured.

In the extraction step, in general, a surfactant for destroying the cell wall of a cell is used. However, when a surfactant is carried over to an ATP measurement reagent containing luciferase, it can be a cause of inhibition of the luciferin-luciferase reaction, for example, by deactivating luciferase. In order to address this problem, luciferases having surfactant resistance have been developed (for example, Patent Literatures 1 and 2). However, there were limitations. Alternatively, in order to address this problem, a dilution step of diluting an extracted solution containing the surfactant carried over therein was employed in some cases.

### Citation List

### Patent Literature

Patent Literature 1: JP Patent Publication (Kohyo) No. 2004-528024 A
Patent Literature 2: JP Patent Publication (Kokai) No. 11-239493 A (1999)

### Non Patent Literature

Non Patent Literature 1: Anal Biochem, 312 (2003) pp. 48-56

### Summary of Invention

### Technical Problem

An object of the present invention is to provide a method and a kit for detecting a microorganism or cell, or detecting a microorganism-related substance or a cell-related substance, which solves at least part of the problems above.

### Solution to Problem

In consideration of the problems above, the present inventors have made earnest studies on an ATP measurement method, resulting in finding the following: When an extraction step is performed priorly as a first step, and the resultant is contacted with a surfactant adsorbent as a second step, surprisingly, ATP can be extracted from a microorganism or cell with an extracting agent, and therefore, ATP can be detected in the sample contacted with the surfactant adsorbent. As a result, the present invention including this finding as an embodiment has been completed.

Specifically, the present invention includes the following embodiments:
[1] A method for detecting a microorganism or cell, comprising the steps of: (i) extracting an adenine nucleotide component of the microorganism or cell by bringing an extracting agent containing a surfactant into contact with a solution that may contain the microorganism or cell without bringing any surfactant adsorbent into contact with the solution that may contain the microorganism or cell; (ii) bringing an analyte comprising the adenine nucleotide component derived from the microorganism or cell, resulting from the extraction of step (i), into contact with a surfactant adsorbent; (iii) bringing the sample contacted with the surfactant adsorbent into contact with a luminescence reagent comprising luciferase and luciferin, or suspending the sample contacted with the surfactant adsorbent in a solution and then bringing the solution suspending the sample into contact with a luminescence reagent comprising luciferase and luciferin; and (iv) measuring luminescence level.
[2] The method according to embodiment 1, wherein the surfactant adsorbent comprises cotton, rayon, cellulose, or polyester.
[3] The method according to embodiment 2, wherein the extracting agent comprises one or more of a surfactant, a cationic surfactant, an anionic surfactant, an amphoteric surfactant, a nonionic surfactant, benzalkonium chloride and n-tetradecyl-N,N-dimethyl-3-ammonio-1-propanesulfonate.
[4] The method according to embodiment 3, wherein the method is a method for detecting a microorganism or cell, the extracting agent is an extracting agent for a microorganism or an extracting agent for a cell for extracting ATP from the microorganism or cell, the adenine nucleotide is ATP, and step (iii) includes allowing ATP to cause luminescence by luciferase.
[5] The method according to embodiment 4, wherein the luminescence reagent further comprises an enzyme that produces ATP from AMP.
[6] The method according to embodiment 4, wherein the luminescence reagent further comprises an enzyme that produces ATP from ADP.
[7] The method according to embodiment 4, wherein the luminescence reagent further comprises an enzyme that produces ATP from AMP, and an enzyme that produces ATP from ADP.
[8] The method according to embodiment 4, wherein the luminescence reagent further comprises an enzyme that produces ADP from AMP, an enzyme that produces ADP from ATP and AMP, and an enzyme that produces ATP from ADP.
[9] The method according to embodiment 4, wherein the luminescence reagent further comprises an enzyme that produces ATP from AMP, and an enzyme that produces AMP from ADP.
[10] The method according to embodiment 1, further comprising, prior to step (i), step (o) of filtering a solution to be tested through a filter to give the solution that may contain the microorganism or cell.
[11] The method according to embodiment 10, wherein the filter is a cellulose acetate filter.
[12] A kit for detecting a microorganism or cell, comprising: (i) an extracting agent comprising a surfactant; (ii) a sampling portion comprising a surfactant adsorbent; (iii) a luminescence reagent comprising luciferase and luciferin; and (iv) instructions for use, wherein the instructions for use states detection of the microorganism or cell by the steps of: (A) extracting an adenine nucleotide component of the microorganism or cell by bringing the extracting agent comprising the surfactant into contact with a solution that may contain the microorganism or cell without bringing any surfactant adsorbent into contact with the solution that may contain the microorganism or cell; (B) collecting an analyte comprising the adenine nucleotide component derived from the microorganism or cell, resulting from the extracting of step (A), by using the sampling portion comprising a surfactant adsorbent; (C) bringing the sample collected with the sampling portion into contact with the luminescence reagent comprising luciferase and luciferin; and (D) measuring luminescence level.
[13] The kit according to embodiment 12, wherein the surfactant adsorbent comprises cotton, rayon, cellulose, or polyester.
[14] The kit according to embodiment 13, wherein the extracting agent comprises one or more of a surfactant, a cationic surfactant, an anionic surfactant, an amphoteric surfactant, a nonionic surfactant, benzalkonium chloride and n-tetradecyl-N,N-dimethyl-3-ammonio-1-propanesulfonate.
[15] The kit according to embodiment 12, wherein the kit is a kit for detecting a microorganism or cell, the extracting agent is an extracting agent for a microorganism or an extracting agent for a cell for extracting ATP from the microorganism or cell, the adenine nucleotide is ATP, and step (C) includes allowing ATP to cause luminescence by luciferase.
[16] The kit according to embodiment 15, wherein the luminescence reagent further comprises an enzyme that produces ATP from AMP.
[17] The kit according to embodiment 15, wherein the luminescence reagent further comprises an enzyme that produces ATP from ADP.
[18] The kit according to embodiment 15, wherein the luminescence reagent further comprises an enzyme that produces ATP from AMP, and an enzyme that produces ATP from ADP.
[19] The kit according to embodiment 15, wherein the luminescence reagent further comprises an enzyme that produces ADP from AMP, an enzyme that produces ADP from ATP and AMP, and an enzyme that produces ATP from ADP.
[20] The kit according to embodiment 15, wherein the luminescence reagent further comprises an enzyme that produces ATP from AMP, and an enzyme that produces AMP from ADP.
[21] The kit according to embodiment 12, further comprising a filter.
[22] The kit according to embodiment 21, wherein the filter is a cellulose acetate filter.

The present specification encompasses the contents disclosed in Japanese Patent Application No. 2022-116858, to which the present application claims priority.

### Advantageous Effects of Invention

As an effect of the present invention, a microorganism can be easily detected.

### Brief Description of Drawings

[Figure 1] Figure 1 illustrates relative luminescence levels in using benzalkonium chloride at various concentrations.
[Figure 2] Figure 2 illustrates relative luminescence levels in using n-tetradecyl-N,N-dimethyl-3-ammonio-1-propanesulfonate at various concentrations.

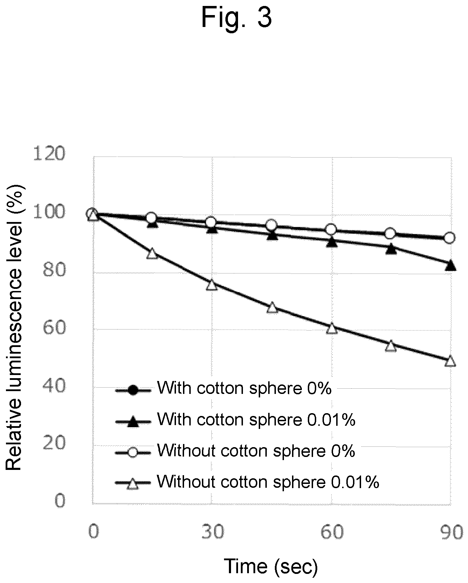

[Figure 3] Figure 3 illustrates change of luminescence level over time in using n-tetradecyl-N,N-dimethyl-3-ammonio-1-propanesulfonate at various concentrations when a cotton ball is used and is not used. In a solution not containing n-tetradecyl-N,N-dimethyl-3-ammonio-1-propanesulfonate (0%), the luminescence level only slightly decreases through consumption of ATP, however, in a solution containing 0.01% n-tetradecyl-N,N-dimethyl-3-ammonio-1-propanesulfonate, the luminescence level greatly decreases when a cotton ball is not contacted. In contrast, even in the presence of 0.01% n-tetradecyl-N,N-dimethyl-3-ammonio-1-propanesulfonate, the decrease is remarkably suppressed when a cotton ball is contacted. Black circles are plotted to overlay white circles in the same positions.
[Figure 4] Figure 4 illustrates the effect of suppressing inhibition of luminescence of benzalkonium chloride with various cotton swabs. LuciPac (Registered trademark) cotton swab (A), industrial cotton swab P1502E (B), industrial cotton swab S-S-P (C), and Mentip (Registered trademark) 1PW1505P (D) are used in the stated order in the rightward direction.
[Figure 5] Figure 5 illustrates the effect of suppressing inhibition of luminescence of n-tetradecyl-N,N-dimethyl-3-ammonio-1-propanesulfonate with various cotton swabs. LuciPac (Registered trademark) cotton swab (A) is used in the left, and industrial cotton swab P1502E (B) is used in the right.
[Figure 6] Figure 6 illustrates change of color tone in a benzalkonium chloride solution contacted with various cotton swabs. Blue color is exhibited at 0.01% and 0.005%, red purple is exhibited at 0%, and an intermediate color tone therebetween is exhibited at 0.0001 and 0.00005%. When S-S-P and LuciPac (Registered trademark) are used, a color tone closer to violet than that observed at 0.001% is exhibited.
[Figure 7] Figure 7 illustrates an example of procedures of microorganism detection by ATP measurement. This is the outline of Example 1.
[Figure 8] Figure 8 illustrates relative luminescence levels obtained in adenine nucleotide measurement of various microorganisms.
[Figure 9] Figure 9 illustrates relative luminescence levels obtained in adenine nucleotide measurement of various microorganisms using an ATP elimination reagent.
[Figure 10] Figure 10 illustrates luminescence levels of various microorganisms (ATP+AMP measurement, filter of Advantec Toyo Inc.).
[Figure 11] Figure 11 illustrates luminescence levels of various microorganisms (ATP+AMP measurement, filter of GVS Filter Technology).
[Figure 12] Figure 12 illustrates luminescence levels of various microorganisms (ATP+AMP measurement, filter of Merck Millipore).
[Figure 13] Figure 13 illustrates luminescence levels of *E. coli* (ATP+ADP+AMP measurement, filter of Advantec Toyo Inc.).
[Figure 14] Figure 14 illustrates luminescence levels of *E. coli* (ATP+ADP+AMP measurement, filter of GVS Filter Technology).
[Figure 15] Figure 15 illustrates luminescence levels of *E. coli* (ATP+ADP+AMP measurement, filter of Membrane Solutions).
[Figure 16] Figure 16 illustrates luminescence levels of *E. coli* (ATP+ADP+AMP measurement, filter of Merck Millipore).

### Description of Embodiments

In a certain embodiment, the present invention provides a method for detecting a microorganism or cell. This method may comprise:
(i) a first step of extracting an adenine nucleotide component of the microorganism or cell by contacting an extracting agent (extracting agent) containing a surfactant with a solution that may contain the microorganism or cell without contacting any surfactant adsorbent with the solution that may contain the microorganism or cell;
(ii) a second step of collecting an analyte comprising the adenine nucleotide component derived from the microorganism or cell, after carrying out the extraction of step (i), with a sampling portion comprising a surfactant adsorbent;
(iii) a third step of contacting the analyte collected with the sampling portion with a luminescence reagent, or suspending the analyte collected with the sampling portion in a solution and then contacting the solution suspending the analyte with a luminescence reagent; and
(iv) a fourth step of measuring luminescence level.

The second step is performed after the first step. The adenine nucleotide may be ATP, ADP, or AMP. In the present specification, the adenine nucleotides, ATP, ADP, and AMP may be referred to as a microorganism-related substance or a cell-related substance.

In a certain embodiment, the surfactant adsorbent may be a surfactant adsorbent that comprise cotton, rayon, cellulose, or polyester. In a certain embodiment, the surfactant adsorbent is selected from the group consisting of cotton, rayon, cellulose, polyester, and a combination thereof. In a certain embodiment, the surfactant adsorbent comprises cotton, rayon, cellulose, or polyester. In a certain embodiment, the surfactant adsorbent is a material that is capable of adsorbing a surfactant, and does not inhibit measurement of an adenine nucleotide (for example, ATP, ADP, or AMP). A surfactant may inhibit a luciferin-luciferase reaction, for example, deactivate luciferase when carried over to an ATP measurement reagent containing luciferase. In the present specification, the phrase (surfactant adsorbent) "adsorbing a surfactant (adsorbs a surfactant)" refers to adsorbing a surfactant to an extent in which deactivation of luciferase or inhibition of a luciferin-luciferase reaction by the surfactant is substantially reduced. Accordingly, a minute amount of adsorption to an extent in which deactivation of luciferase or inhibition of a luciferin-luciferase reaction by the surfactant is not substantially affected (for example, usual physical adsorption of the surfactant in a very small amount on a container surface) shall not be encompassed by "adsorbing a surfactant" as used herein. In other words, a material that adsorbs only a very small amount of a surfactant to extent in which deactivation of luciferase or inhibition of a luciferin-luciferase reaction by the surfactant is not substantially affected shall not be encompassed by the surfactant adsorbent of the present disclosure (such material is excluded from the surfactant adsorbent of the present disclosure). The sampling portion comprising a surfactant adsorbent may be of any shape. In a certain embodiment, the sampling portion comprising a surfactant adsorbent can be in the shape of a thin rod, for example, in the shape of a cotton swab, or a cotton ball, and the shape is not limited thereto. When the sampling portion comprising a surfactant adsorbent is in the shape of a cotton swab, the surfactant adsorbent may be disposed at an end (tip end) of the cotton swab.

In a certain embodiment, the luminescence reagent may contain luciferase and luciferin.

In a certain embodiment, the extracting agent may contain one or more of a surfactant, a cationic surfactant, an anionic surfactant, an amphoteric surfactant, a nonionic surfactant, and benzalkonium chloride.

In a certain embodiment, the method is a method for detecting a microorganism. In this case, the extracting agent may be an extracting agent for a microorganism for extracting ATP from a microorganism, the adenine nucleotide is ATP, and step (iii) may comprise allowing ATP to cause luminescence by luciferase. In another embodiment, the method is a method for detecting a cell. In this case, the extracting agent is an extracting agent for a microorganism for extracting ATP from a cell, the adenine nucleotide is ATP, and step (iii) may comprise allowing ATP to cause luminescence by luciferase.

In the present specification, a microorganism and a cell may be together referred to, for convenience, as an analyte from which ATP or the like contained therein is to be taken out by extraction. In a certain embodiment, the sample from which ATP or the like contained therein is to be taken out by extraction comprises a cell. In another embodiment, the sample from which ATP or the like contained therein is to be taken out by extraction comprises a microorganism. The terms "microorganism" and "cell" are not mutually exclusive. An analyte (i.e., what is being measured) that is a microorganism and a cell at the same time can exist (for example, a unicellular microorganism). When mutually exclusive terms should be used, the sample from which ATP or the like contained therein is to be taken out by extraction comprises a microorganism, and a non-microorganism cell. Examples of non-microorganism cells (non-microbial cells) include cells not derived from microorganisms, such as an animal cell, an animal-derived cell, a plant cell, a plant-derived cell, an insect cell, and an insect-derived cell and the like. In the present specification, true fungi and fungi are regarded as microorganisms for convenience.

In a certain embodiment, the luminescence reagent can further contain an enzyme that generates ATP from AMP. In a certain embodiment, the luminescence reagent can further contain an enzyme that generates ATP or AMP from ADP.

In a certain embodiment, the method can comprise, before step (i), step (o) of filtering the solution to be tested through a filter to collect the microorganism or cell on the filter. The filter may be used, for example, to remove an impurity, or to concentrate the microorganism or cell. Unless specified otherwise, The filter collects the microorganism or cell thereon and allows impurity to pass therethrough as a filtrate. In a certain embodiment, a filter having a pore size capable of collecting the microorganism or cell is used, and a filter having, for example, a pore size of 0.2 µm or more, for example, 0.2 µm, 0.45 µm, 0.5 µm, 0.8 µm, or 1 µm can be used. For example, a membrane filter unit for filtration that can be attached to a syringe or the like can be used. Examples of the filter material include, but are not limited to, a cellulose mixed ester, cellulose acetate, polyether sulfone (PES), polyvinylidene fluoride (PVDF), hydrophilic polytetrafluoroethylene (PTFE), PTFE, and nitrocellulose and the like. In a certain embodiment, the filter can be a filter containing cellulose acetate. In a certain embodiment, the filter is a cellulose acetate filter. A cellulose mixed ester film is known as a mixed cellulose ester film containing cellulose acetate and nitrocellulose. However, in the present specification, the term "cellulose acetate filter" means a filter consisting of cellulose acetate. Moreover, a filter of a cellulose mixed ester film and a filter comprising a cellulose mixed ester film shall not be encompassed by the "cellulose acetate filter" as used herein.

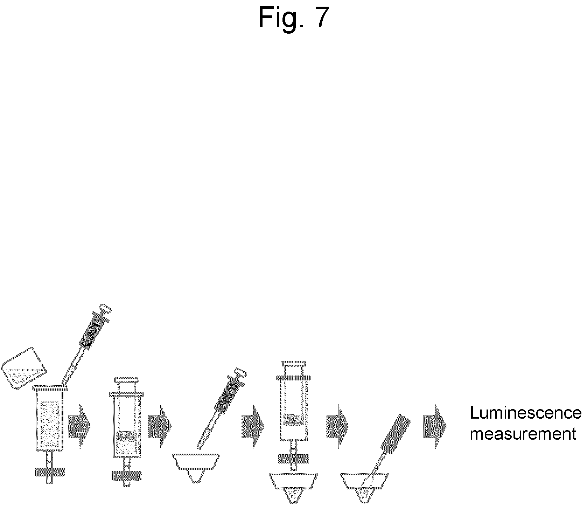

To apply the extracting agent to the microorganism or cell collected on the filter, for example, with the filter connected to the syringe used for the filtration, the extracting agent may be drawn into a syringe to perform extraction, and then the drawn solution may be discharged from the syringe. See, for example, Figure 7.

In a certain embodiment, the present invention provides a kit for performing the above-described method. In a certain embodiment, the kit may comprise:
(i) an extracting agent comprising a surfactant;
(ii) a sampling portion comprising a surfactant adsorbent;
(iii) a luminescence reagent; and
(iv) instructions for use.
The instruction may state procedures for performing the method. In a specific embodiment, the kit may further comprise a luminescence level measurement portion. In another embodiment, the kit may be used in combination with a luminescence level measurement portion. Examples of the luminescence level measurement portion include, but are not limited to, commercially available apparatus for measuring luminescence such as Lumitester (Registered trademark) C-110, and Lumitester (Registered trademark) Smart.

With regard to the kit, the surfactant adsorbent may comprise cotton, rayon, cellulose, or polyester. In a certain embodiment, examples of the surfactant adsorbent comprised in the kit include cotton, rayon, cellulose, and polyester. In a certain embodiment, the surfactant adsorbent comprised in the kit is selected from the group consisting of cotton, rayon, cellulose, polyester, and a combination thereof. In a certain embodiment, the surfactant adsorbent comprised in the kit consists of cotton, rayon, cellulose, or polyester. The luminescence reagent may comprise luciferase and luciferin. The extracting agent may comprise one or more of a surfactant, a cationic surfactant, an anionic surfactant, an amphoteric surfactant, a nonionic surfactant, or benzalkonium chloride and n-tetradecyl-N,N-dimethyl-3-ammonio-1-propanesulfonate.

In a certain embodiment, the kit is a kit for detecting a microorganism or cell, the extracting agent is an extracting agent for a microorganism or an extracting agent for a cell for extracting ATP from a microorganism or cell, the adenine nucleotide is ATP, and step (iii) may comprise allowing ATP to cause luminescence by luciferase. With regard to the kit, the luminescence reagent may further comprise an enzyme that generates ATP from AMP. With regard to the kit, the luminescence reagent may further comprise an enzyme that generates ATP or AMP from ADP. Moreover, the kit may further comprise a filter. The filter may be a filter containing cellulose acetate. The filter may be a cellulose acetate filter.

### Extracting agent

The extracting agent contains a surfactant. The extracting agent may contain cell lysis reagents such as lysozyme (EC 3.2.1.17), and a combination thereof. In a certain embodiment, examples of the surfactant usable in the extracting agent include a nonionic surfactant, a cationic surfactant, an anionic surfactant, and a zwitterionic surfactant, such as benzalkonium chloride or n-tetradecyl-N,N-dimethyl-3-ammonio-1-propanesulfonate (Zwittergent (Registered trademark) 3-14, CAS14933-09-6). Examples of the nonionic surfactant include, but are not limited to, polyoxyethylene alkyl ether, fatty acid sorbitan ester, alkyl polyglucoside, fatty acid diethanolamide, and alkyl monoglyceryl ether, such as Triton (Trademark) X-100, Triton (Trademark) X-45, Tween (Registered trademark) 20, Tween (Registered trademark) 60, Tween (Registered trademark) 80, Span (Registered trademark) 60, Span (Registered trademark) 80, and Thesit (Registered trademark). Examples of the cationic surfactant include a quaternary ammonium salt, a pyridinium salt, a phosphonium salt, such as an alkyl trimethyl ammonium salt, a dialkyl dimethyl ammonium salt, an alkyl benzyl dimethyl ammonium salt, an alkyl pyridinium salt, an alkyl phosphonium salt, an imidazolium salt, an alkyl imidazolium salt, an isoquinonium salt, an alkyl isoquinonium salt, cetyltrimethylammonium or benzyldimethyldodecylammonium bromide, and benzalkonium chloride and the like. Examples of the quaternary ammonium salt include decyltrimethylammonium chloride or bromide, dodecyltrimethylammonium chloride or bromide, tetradecyltrimethylammonium chloride or bromide, hexadecyltrimethylammonium chloride or bromide, octadecyltrimethylammonium chloride or bromide, and eicosyltrimethylammonium chloride or bromide. Examples of the pyridinium salt include 1-decylpyridinium chloride or bromide, 1-dodecylpyridinium chloride or bromide, 1-tetradecylpyridinium chloride or bromide, 1-hexadecylpyridinium chloride or bromide, N-cetyl-2-methylpyridinium chloride, N-cetyl-3-methylpyridinium chloride, N-cetyl-4-methylpyridinium chloride, 1-octadecylpyridinium chloride or bromide, and 1-eicosylpyridinium chloride or bromide. Examples of the phosphonium salt include tetraethylphosphonium chloride or bromide, tributylmethylphosphonium chloride, bromide, or iodide, tetrabutylphosphonium chloride or bromide, tetra-n-octylphosphonium chloride or bromide, tributyldodecylphosphonium chloride or bromide, tributylhexadecylphosphonium chloride or bromide, methyltriphenylphosphonium chloride, bromide, or iodide, and tetraphenylphosphonium chloride or bromide. Examples of the zwitterionic surfactant include, but are not limited to, sulfobetaine-based surfactants, such as sulfobetaine 3-10, alkyldimethylamine oxide, alkylcarboxybetaine, CHAPS, CHAPSO, Big Chap, Brij (Registered trademark), Zwittergent (Registered trademark) 3-14 (3-(N,N-dimethylmyristylammonio)propanesulfonate). Examples of the anionic surfactant include deoxycholate salt, linear alkylbenzene sulfonate salt, alkyl sulfate salt, alpha-olefin sulfonate salt, polyoxyethylene alkyl ether sulfate salt, α-sulfo fatty acid ester salt, and an alkali metal salt of a natural fatty acid, such as sodium dodecyl sulfate (SDS).

In a certain embodiment, when the surfactant contained in the extracting agent is adsorbed by the surfactant adsorbent contained in the sampling portion, the amount of the surfactant contained in the mixture of the sample collected by the sampling portion and the luminescence reagent is reduced. Here, the phrase "the amount of the surfactant contained in the mixture of the sample collected by the sampling portion and the luminescence reagent is reduced" refers to the amount of the surfactant contained in the mixture of the sample collected by the sampling portion and the luminescence reagent being reduced to an extent in which an enzyme for measuring ATP contained in the luminescence reagent, for example, a reporter molecule such as luciferase, is not harmfully affected, is not substantially harmfully affected, or is not significantly reduced in activity thereof, or the influence on a reporter molecule such as luciferase is reduced. Here, the phrase "not substantially harmfully affected", "not significantly reduced in the activity thereof", or "reduce the influence" refers to ATP measurement not being affected, or can be performed as a whole even if affected. In other words, the extracting agent may contain any amount of surfactant as long as the surfactant is adsorbed by the surfactant adsorbent contained in the sampling portion, and thus the ATP measurement reaction with the luminescence reagent is not reduced as a result. The surfactant contained in the extracting agent may be contained in the extracting agent in an amount corresponding to the concentration of the surfactant in the measurement system of 0.0001% by weight to 5% by weight, 0.001% by weight to 3% by weight, 0.01% by weight to 2% by weight, or 0.1% by weight to 1.5% by weight.

The luminescence reagent may also comprise an enzyme-stabilizing agent such as bovine serum albumin or gelatin that protects a reporter molecule such as luciferase from degradation. A substance that adjusts pH or improves preservation may also be added to the luminescence reagent. Examples thereof include appropriate pH buffers (HEPES, Tricine, Tris, phosphate buffer solutions, acetate buffer solutions, and the like), reducing agents (dithiothreitol (DTT), 2-mercaptoethanol, and the like), and sugars (glucose, sucrose, trehalose, and the like).

### Luminescence Reagent

In a certain embodiment, the luminescence reagent comprises luciferase and luciferin. In this case, a metal ion such as magnesium, manganese, or calcium may also be included. ATP, O₂, and luciferin are converted by luciferase into AMP, pyrophosphoric acid, CO₂, and oxyluciferin, and luminescence is generated during the conversion. The luciferase may be a luciferase found in nature or may be a genetically engineered recombinant luciferase mutant. The luciferase mutant may be generated by site-directed mutagenesis or by random mutagenesis. The luciferase mutant may be a fusion protein with a protein having another function. The luciferase mutant may have desired properties such as improved heat resistance, or improved surfactant resistance and the like.

The luminescence level from luciferase can be evaluated using relative luminescence level (RLU) as an indicator obtained by using an appropriate apparatus for measuring luminescence, for example, a luminometer (Lumat LB9510, Junior LB9509, Sirius 2 LB9526, or Centro LB963 manufactured by Berthold Technologies GmbH & Co. KG; Lumitester (Registered trademark) C-110, and Lumitester (Registered trademark) Smart manufactured by Kikkoman Biochemifa Company, or the like). Typically, luminescence generated during conversion from luciferin to oxyluciferin is measured.

The luciferase is not particularly limited, as long as the substrate thereof is ATP, and those derived from bacteria, protozoans, animals, mollusks, and insects can be used. Examples of luciferase derived from insects include coleopteran luciferase, and examples thereof include luciferase derived from fireflies such as the genus *Photinus,* for example, *Photinus pyralis;* the genus *Photuris,* for example, *Photuris lucicrescens, Photuris pennsylvanica*; the genus *Luciola,* for example, *Luciola cruciata*, *Luciola lateralis*, *Luciola parvula*; the genus *Pyrocoelia*; and *Lucidina biplagiata*; and those derived from click beetle of the genus *Pyrophorus* and the like. Numerous genes for luciferase have been reported, and their nucleotide sequences and amino acid sequences are available from known databases such as GeneBank.

The luciferase gene may be a wildtype gene or a gene having a mutation. The mutation may be a mutation introduced site-specifically or a random mutation. Examples of known mutations include, but are not limited to, mutations that improve the luminescence level as described in JP Patent Publication (Kokai) No. 2011-188787 A; mutations that increase the luminescence durability as described in JP Patent Publication (Kokai) No. 2000-197484 A; mutations that change the luminescence wavelength as described in JP Patent No. 2666561 or JP Patent Publication (Kohyo) No. 2003-512071 A; mutations that increase resistance to surfactant as described in JP Patent Publication (Kokai) No. 11-239493 A (1999); mutations that increase affinity for the substrate as described in International Publication No. WO 99/02697 pamphlet, JP Patent Publication (Kohyo) No. 10-512750 A (1998), or JP Patent Publication (Kohyo) No. 2001-518799 A; and mutations that increase the stability as described in JP Patent No. 3048466, JP Patent Publication (Kokai) No. 2000-197487 A, JP Patent Publication (Kohyo) No. 9-510610 A (1997), and JP Patent Publication (Kohyo) No. 2003-518912 A.

The luciferase gene and a recombinant DNA thereof can be prepared with conventional methods. For example, JP Patent Publication (Kokoku) No. 7-112434 B (1995) describes a luciferase gene from *Luciola lateralis.* Moreover, JP Patent Publication (Kokai) No. 1-51086 A (1989) describes a luciferase gene from *Luciola cruciata.*

The luciferase gene can be incorporated into a vector such as a plasmid, a bacteriophage, or a cosmid and the like, with which an appropriate host may be transformed or transfected. The host may be a microorganism, a bacterium such as *Escherichia coli,* yeast, or the like. The transformed host having luciferase-producing ability can be cultured with various known methods.

Examples of the medium include those obtained by adding, to one or more nitrogen sources such as triptone, yeast extract, meat extract, peptone, corn steep liquor, or an exudate of soybean or wheat bran; one or more inorganic salts such as sodium chloride, potassium dihydrogen phosphate, dipotassium phosphate, magnesium chloride, ferric chloride, magnesium sulfate, or manganese sulfate and the like, and if necessary carbohydrate raw materials, vitamins, and the like.

The initial pH of the medium can be, for example, 7 to 9. The culture can be conducted, for example, at 30 to 40°C for 2 to 24 hours, by aerated and agitated culture, shaking culture, static culture, or the like. After culturing, the luciferase is collected from the culture by a known technique.

More specifically, luciferase is extracted by subjecting the bacterial cells to an ultrasonic homogenization treatment, a grinding treatment, or the like in a conventional way or using a lytic enzyme such as lysozyme. A crude enzyme can be obtained by treating the resultant extract by filtration, centrifugation, or the like, if necessary, removing nucleic acid with streptomycin sulfate or the like, and adding ammonium sulfate, alcohol, acetone, or the like to the same.

The crude enzyme may further be purified with various techniques such as gel filtration and chromatography. Commercially available luciferase can also be used and, for example, the luciferase of Kikkoman Biochemifa Company, cat. No. 61314 can be used. This luciferase is a luciferase described in JP Patent Publication (Kokai) No. 11-239493 A (1999) (JP Patent No. 3749628), and the amino acid sequence thereof is shown in SEQ ID NO: 1 herein. Moreover, commercially available luciferase from Sigma Aldrich, Promega KK., Molecular Probes (Registered trademark) of Life Technology Inc. can also be used.

The luciferin may be any luciferin as long as it is recognized as a substrate by the luciferase being used, and may be natural or chemically synthesized. Moreover, any known luciferin derivative may also be used. The basic structure of luciferin is imidazopyrazinone, and there are many tautomers thereof. Examples of the luciferin include, but are not limited to, firefly luciferin, bacterial luciferin, vargulin, dinoflagellate luciferin, and coelenterazine. The firefly luciferin is a substrate of firefly luciferase (EC 1.13.12.7). The bacterial luciferin is found in bacteria and fish, and contains a long-chain aldehyde and reduced riboflavin phosphate. Vargulin is an imidazolopyrazine derivative found in midshipman fish and ostracods. The dinoflagellate luciferin is a chlorophyll derivative. Coelenterazine is found in radiolaria, ctenophore, cnidarians, squid, chaetognaths, copepods, shrimp, fish and the like. Another example includes latial luciferin of *Latia neritoides,* (E)-2-methyl-4-(2,6,6-trimethyl-1-cyclohex-1-yl)-1-buten-1-ol formic acid. The luciferin derivative may be those described in JP Patent Publication (Kokai) No. 2007-91695 A, JP Patent Publication (Kohyo) No. 2010-523149 A (International Publication No. 2008/127677), and the like.

In a certain embodiment, the final concentration (mg protein/mL) of luciferase in the measurement system can be 0.001 µg protein/mL or more, 0.01 µg protein/mL or more, 0.02 µg protein/mL or more, 0.05 µg protein/mL or more, 0.10 µg protein/mL or more, 0.20 µg protein/mL or more, 0.25 µg protein/mL or more, 0.5 µg protein/mL or more, 0.75 µg protein/mL or more, 1 µg protein/mL or more, 5 µg protein/mL or more, 10 µg protein/mL or more, 20 µg protein/mL or more, 30 µg protein/mL or more, 40 µg protein/mL or more, 50 µg protein/mL or more, 60 µg protein/mL or more, 70 µg protein/mL or more, 80 µg protein/mL or more, 90 µg protein/mL or more, 100 µg protein/mL or more, 110 µg protein/mL or more, 120 µg protein/mL or more, 130 µg protein/mL or more, 140 µg protein/mL or more, or for example, 150 µg protein/mL or more when absorbance at 280 nm is defined as the luciferase concentration. In a certain embodiment, the final concentration (mg protein/mL) of luciferase in the measurement system can be 200 µg protein/mL or less, 150 µg protein/mL or less, 140 µg protein/mL or less, 130 µg protein/mL or less, 120 µg protein/mL or less, 110 µg protein/mL or less, 100 µg protein/mL or less, 90 µg protein/mL or less, 80 µg protein/mL or less, 70 µg protein/mL or less, 60 µg protein/mL or less, 50 µg protein/mL or less, 40 µg protein/mL or less, 30 µg protein/mL or less, 20 µg protein/mL or less, 10 µg protein/mL or less, 5 µg protein/mL or less, 1 µg protein/mL or less, 0.5 µg protein/mL or less, or 0.3 µg protein/mL or less when absorbance at 280 nm is defined as the luciferase concentration. In a certain embodiment, the final concentration of luciferin or a luciferin derivative in the measurement system can be 0.01 mM to 20 mM, 0.02 mM to 20 mM, 0.03 mM to 20 mM, 0.04 mM to 20 mM, 0.05 mM to 20 mM, 0.1 mM to 20 mM, or 0.5 mM to 10 mM, and for example, 0.75 mM to 5 mM.

### Enzyme that Produces ATP from AMP

In a certain embodiment, the luminescence reagent may further comprise an enzyme that produces ATP from AMP. With this enzyme, not only ATP but also AMP can be measured as an adenine nucleotide present in the system. Examples of the enzyme that produces ATP from AMP include, but are not limited to, pyruvate-phosphate dikinase (PPDK), and pyruvate-water dikinase (PWDK) and the like. For example, PPDK may be used together with phosphoenolpyruvic acid (PEP) and phosphoric acid. For example, PWDK may be used together with PEP and water. In another embodiment, a plurality of enzymes may be used in combination.

### Enzyme that Produces ATP from ADP

In a certain embodiment, the luminescence reagent may further comprise an enzyme that produces ATP from ADP. With this enzyme, not only ATP but also ADP can be measured as an adenine nucleotide present in the system. Examples of the enzyme that produces ATP from ADP include, but are not limited to, pyruvate kinase (PK), creatine kinase, acetate kinase, polyphosphate kinase, hexokinase, glucokinase, glycerol kinase, fructokinase, phosphofructokinase, riboflavin kinase, and fructose-bisphosphatase.

### Enzyme that Produces AMP from ADP

In a certain embodiment, the luminescence reagent may further comprise an enzyme that produces AMP from ADP. For example, when an enzyme that produces AMP from ADP and an enzyme that produces ATP from AMP are combined with luciferase, AMP, ADP, and ATP can be measured. Examples of the enzyme that produces AMP from ADP include, but are not limited to, ADP-dependent hexokinase and apyrase and the like.

### Enzyme that Produces ADP from AMP

In a certain embodiment, the luminescence reagent may further comprise an enzyme that produces ADP from AMP. For example, when an enzyme that produces ADP from AMP and an enzyme that produces ATP from ADP are combined with luciferase, AMP, ADP, and ATP can be measured. An example of the enzyme that produces ADP from AMP includes, but is not limited to, ADP-dependent hexokinase and the like. Although the ADP-dependent hexokinase usually produces AMP from ADP, it can also catalyze the reverse reaction, and therefore, can be utilized as an enzyme that produces ADP from AMP by appropriately adjusting the substrate concentration.

### Enzyme that Produces ADP from ATP and AMP

In a certain embodiment, the luminescence reagent may further comprise an enzyme that produces ADP from ATP and AMP. For example, when an enzyme that produces ADP from ATP and AMP and an enzyme that produces ATP from ADP are combined, AMP, ADP, and ATP can be measured. An example of the enzyme that produces ADP from ATP and AMP includes, but is not limited to, adenylate kinase (AK).

The enzyme that produces ATP from AMP and the enzyme that produces ATP from ADP may collectively be referred to as an enzyme having ATP-producing ability. As the enzyme having ATP-producing ability, any known enzyme can be used, and examples thereof include, but are not limited to, kinases having ATP-producing ability. Examples of kinases having ATP-producing ability include, but are not limited to, pyruvate kinase, pyruvate-phosphate dikinase, creatine kinase, acetate kinase, polyphosphate kinase, hexokinase, glucokinase, glycerol kinase, fructokinase, phosphofructokinase, riboflavin kinase, fructose-bisphosphatase, and combinations thereof.

Pyruvate kinase (EC 2.7.1.40) converts phosphoenolpyruvate into pyruvate in glycolysis, and ADP is converted into ATP during this conversion. This reaction is an exergonic reaction, where the change in the Gibbs energy is negative, and irreversible under natural conditions:

PEP + ADP → pyruvate + ATP

The reverse reaction is catalyzed by pyruvate carboxylase and phosphoenolpyruvate carboxykinase in gluconeogenesis and produces PEP and ADP from ATP and pyruvic acid. When cell extraction is performed, various enzymes are mixed in the system and the aforementioned reactions can progress in the both directions. In the system, if phosphoenolpyruvate is present at high concentrations, ADP can be converted into ATP. Moreover, if not only phosphoenolpyruvate but also pyruvate kinase is present in the system, it is considered that more ADP is converted into ATP.

Creatine kinase (EC 2.7.3.2) mediates the conversion reaction from creatine and ATP to creatine phosphate and ADP and vice versa:

Creatine + ATP ←→ creatine phosphate + ADP

Creatine kinase (CK) is also referred to as creatine phosphokinase (CPK) or phosphocreatine kinase. In the present specification, these terms are mutually interchangeable. In muscles and the like of animals, creatine phosphate and ADP are usually produced from creatine and ATP. However, this reaction is a reversible reaction, and if creatine phosphate and ADP are present in the system at high concentrations, the reaction may progress in the reverse direction to produce creatine and ATP. In the living body, cytoplasmic creatine kinase is composed of two subunits of B or M. Therefore, the 3 isozymes CK-MM, CK-BB, and CK-MB can be present depending on the combination of the subunits. While the isozyme pattern differs depending on the tissue, any combination can be used in the present invention.

The pyruvate-phosphate dikinase (EC 2.7.9.1) catalyzes the reaction between ATP, pyruvate, and orthophosphate, and adenosine monophosphate (AMP), phosphoenolpyruvate (PEP), and pyrophosphate (PPi):

ATP + pyruvate + phosphate ←→ AMP + PEP + PPi

The pyruvate-phosphate dikinase (PPDK) is also referred to as ATP:pyruvate, phosphate phosphotransferase, pyruvate orthophosphate dikinase, and pyruvate phosphate ligase. In the present specification, these terms are mutually interchangeable. PPDK, in general, converts pyruvate into PEP, and 1 molecule of ATP is consumed and converted into AMP in the process. The reaction is divided into the following 3 reversible reactions.
1. The enzyme PPDK binds to ATP, converts the same into AMP, and produces diphosphorylated PPDK.
2. The diphosphorylated PPDK binds to inorganic phosphate and produces diphosphate and monophosphorylated PPDK.
3. The monophosphorylated PPDK binds to pyruvate and produces PEP and reproduces PPDK.

In the reactions, if the concentration of PEP present in the system is high, reactions progress in the reverse direction as follows.

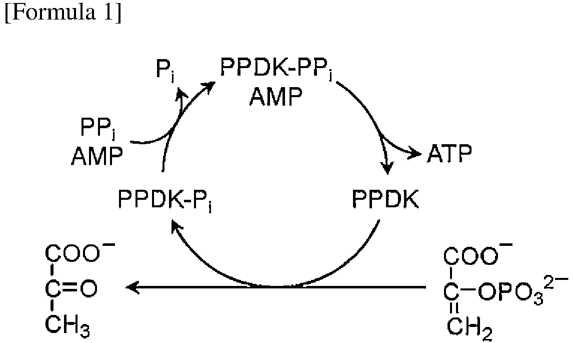

For convenience, the reaction steps are described with the same number as those described above.
3. PEP binds to PPDK and produces monophosphorylated PPDK and pyruvate.
2. From the diphosphate and monophosphorylated PPDK, diphosphorylated PPDK and inorganic phosphate are produced.
1. From the diphosphorylated PPDK and AMP, PPDK and ATP are produced.

Acetate kinase (EC 2.7.2.1) catalyzes conversion from ATP and acetate to ADP and acetylated phosphate and vice versa in the presence of a cation:

ATP + acetate ←→ ADP + acetylated phosphate

Acetate kinase (AK) is also referred to as ATP:acetate phosphotransferase or acetyl kinase. In the present specification, these terms are mutually interchangeable. In the living body, ADP and acetylated phosphate are produced from ATP and acetate, and eventually promote reactions to produce acetyl CoA. If acetylated phosphate and ADP produced from acetyl CoA are present in the system, these can be converted into acetate and ATP.

Polyphosphate kinase (EC 2.7.4.1) catalyzes the reaction to convert polyphosphate (PolyPₙ) and ADP into polyphosphate (PolyPₙ₋₁) and ATP:

ADP + PolyPₙ ←→ ATP + PolyPₙ₋₁

Polyphosphate kinase (PPK) is also referred to as ATP:polyphosphate phosphotransferase. In the present specification, these terms are mutually interchangeable. PPK is involved in oxidative phosphorylation in the living body. If polyphosphate (n) and ADP are present in the system, these can be converted into polyphosphate (n-1) and ATP.

The riboflavin kinase (EC 2.7.1.26) is also described as FMNK and catalyzes the reaction to convert riboflavin and ATP into riboflavin phosphate (FMN) and ADP:

ATP + riboflavin ←→ ADP + FMN

The riboflavin kinase belongs to ATP:riboflavin 5'-phosphotransferase (also referred to as flavokinase).

The phosphofructokinase 1 (EC 2.7.1.11) is also described as PFK1 and catalyzes the reaction to convert fructose-6-phosphate (Fru6P) and ATP into fructose-1,6-bisphosphate (Fru1,6-BP) and ADP:

Fru6P + ATP ←→ Fru1,6-BP + ADP

The phosphofructokinase 1 belongs to phosphofructokinases. As used herein, phosphofructokinase 1 may be described as Fru-1,6BPK.

Fructose-bisphosphatase (EC 3.1.3.11) is also described as FBPase and catalyzes the reaction to convert fructose-1,6-bisphosphate (Fru1,6-BP) and ADP into fructose-6-phosphate (Fru6P) and ATP:

Fru1,6-BP + ADP ←→ Fru6P + ATP

Fructose-bisphosphatase may also be described as FBP, FBP1.

The aforementioned enzymes may be used in the method of the present disclosure.

The enzymes having ATP-producing ability that can be used include any known enzymes such as those derived from microorganisms, bacteria, eukaryotes, protists, plants, and animals, and, for example, a commercially available enzyme can be used. The amount of the enzyme having ATP-producing ability to be added can be set as appropriate depending on the concentration and the reaction system of interest.

Various enzymes having ATP-producing ability are known, which recognizes various substrates, and the present invention focuses on the ATP-producing abilities thereof. Accordingly, as used herein, the activity unit (U) of the enzymes having ATP-producing ability is defined as the amount of the enzyme that converts 1.0 µmole of substrate into ATP per minute at 37°C at pH 7.8 (1 U = 1 µmol ATP/min, pH 7.8, 37°C). In a certain embodiment, the enzymes having ATP-producing ability can be added such that the activity unit in the measurement system is 0.001 U or more, 0.01 U or more, 0.1 U or more, 1 U or more, 2 U or more, 3 U or more, 4 U or more, or 5 U or more. In a certain embodiment, the enzymes having ATP-producing ability can be added such that the activity unit in the measurement system is 1000 U or less, 100 U or less, 50 U or less, 10 U or less, 9 U or less, 8 U or less, 7 U or less, or 6 U or less.

The sample for measurement may comprise a solution that may comprise a cell, a medium that may comprise a cell or a microorganism, for example, tap water, river water, treated river water, beverages, drinking water, industrial water, bathwater, hot spring water, water of a fountain, rinse water for cleaning in place (CIP), industrial wastewater, water of ponds in amusement facilities, water of swimming pool, water to be injected into a water tank, cooling water for a cooling tower, a hemodialysis solution, industrial treated water, liquid pharmaceuticals, supplements, saline, and the like. Examples of the microorganism include prokaryotes, bacteria, yeast, protists, fungi, and the like. Examples of the cell include, in addition to microorganism cells, animal cells, animal-derived cells, plant cells, plant-derived cells, insect cells, and insect-derived cells.

When adenine nucleotides such as ATP, ADP, and AMP in the sample are measured, the presence or absence of a microorganism or cell can be qualitatively or quantitatively determined. This technique can be used for determining contamination of beverages or food products with a microorganism or cell, contamination of water with a microorganism or cell, quantifying propagated microorganisms, quantifying grown cells, sanitary inspection, and the like.

For the ATP measurement, LuciPac (Registered trademark) II (manufactured by Kikkoman Biochemifa Company, Product No.: 60375) may be used, and for the ATP+AMP measurement, LuciPac (Registered trademark) Pen (manufactured by Kikkoman Biochemifa Company, Product No.: 60331) may be used, and for the ATP+ADP+AMP measurement, LuciPac (Registered trademark) A3 Surface (manufactured by Kikkoman Biochemifa Company, Product No.: 60361) may be used. As the extraction reagent, a 0.01% benzalkonium chloride solution, or a 0.01% n-tetradecyl-N,N-dimethyl-3-ammonio-1-propanesulfonate solution may be used. The adenine nucleotides may be measured by a similar method by referring to instructions attached to a commercially available kit of these products.

For the ATP measurement, Lumitester (Registered trademark) C-110 (manufactured by Kikkoman Biochemifa Company) may be used, and for the ATP+AMP measurement and the ATP+ADP+AMP measurement, Lumitester (Registered trademark) Smart (manufactured by Kikkoman Biochemifa Company) may be used. For example, 0.2 mL of sample solutions containing benzalkonium chloride at various concentrations are added respectively to 0.1 mL of an ATP measurement reagent, and 0.01 mL of a 1 × 10⁻⁷ M ATP solution is further added to the resultant, and then, the luminescence level can be measured. Measurement of the luminescence level may be performed using Lumitester (Registered trademark) C-110 (manufactured by Kikkoman Biochemifa Company).

A standard can be defined and the luminescence can be expressed as the relative luminescence unit (RLU) compared thereto.

In a certain embodiment, the method of the present disclosure may comprise a step of eliminating ATP, ADP, and/or AMP present outside the bacterial cell. Moreover, the kit of the present disclosure may comprise a reagent for eliminating ATP, ADP, and/or AMP present outside the bacterial cell. ATP, ADP, and/or AMP may be eliminated with a known reagent or a commercially available reagent. As a method for degrading ATP, when only ATP is to be measured, a method using apyrase is known (see, for example, JP Patent Publication (Kokai) No. 11-89592 A (1999)). However, although apyrase is an enzyme that degrades ATP into ADP, and further into AMP, it does not catalyze further dephosphorylation reaction, and does not function as an elimination reagent in the measurement of ATP+AMP or ATP+ADP+AMP containing an enzyme that produces ATP from AMP. An example of the known reagent includes, but is not limited to, adenosine phosphate deaminase. In Non Patent Literature 1, a method using adenosine phosphate deaminase is performed to eliminate an adenine nucleotide, and ATP degradation requires leaving still for 30 minutes. An example of the commercially available reagent includes, but is not limited to, Lucifer ATP Elimination Reagent (manufactured by Kikkoman Biochemifa Company, Product No.: 60254).

The method of the present disclosure is characterized in that the sample containing the extracting agent and the like is contacted with an extracting agent adsorbent (surfactant adsorbent) such as a cotton swab after the ATP extraction. The same goes with the kit of the present disclosure. There is a known method using a cotton swap or a sampling tool in measuring a microorganism present in water. However, in conventional methods, in general, water is first collected with a cotton swab or a sampling tool, and then, such tool is immersed in an extracting agent to extract ATP from a microorganism, and the thus extracted liquid is added to a luminescence reagent. The cotton swab is used for sampling water, and therefore is used before the ATP extraction step, and the ATP extraction is performed after sampling. To the best of the present inventors' knowledge, there has been no report of a microorganism measurement method in which extraction, for example, ATP extraction is performed first, and then the resultant is contacted with a sampling tool such as a cotton swab. In addition, there has been no report of a microorganism measurement method in which extraction is performed first, and then the resultant is contacted with a surfactant adsorbent. Upon considering adding a sampled reagent to a luminescence reagent, according to common general knowledge, when a step of contacting with a cotton swab or the like after ATP extraction is included, there are concerns that ATP may not be sufficiently collected from the cotton swab, and therefore concerns that the luminescence level may be reduced. Therefore, in general, a solution resulting from extraction is not contacted with a cotton swab or the like but directly subjected to luminescence measurement. On the other hand, when extraction is performed with an extracting agent comprising a surfactant, a sample solution thus extracted contains a considerable amount of the surfactant, which is carried over to the measurement reagent to cause harmful effects such as inhibition of luminescence of a luminescence reagent, and deactivation of a luminescence enzyme. However, The present inventors found that when the step of contacting with a cotton swab after the ATP extraction is included, the recovery rate of adenine nucleotide such as ATP is not substantially reduced, the surfactant can be selectively adsorbed, and therefore, the amount of the surfactant carried over to a mixture with the luminescence reagent can be reduced. In other words, even when an extracted solution is sampled with the sampling portion containing a surfactant adsorbent, extracted ATP could be measured with a luminescence reagent. It is very surprising that a cotton swab can selectively adsorb only the surfactant without affecting the ATP recovery rate.

Adsorption phenomenon is observed for various substances, and for example, an ordinary container surface and an ordinary filter may also physically adsorb a very small amount of a surfactant. However, the amount of the surfactant adsorbed by an ordinary container surface and an ordinary filter is very small, and it is impossible or difficult to suppress, with adsorption in such a very small amount, luciferase deactivation and inhibition of the luciferin-luciferase reaction by the surfactant. Such adsorption in a very small amount is not encompassed by "adsorbing a surfactant" as used herein. For example, in a certain embodiment, before step (i) of extracting an adenine nucleotide component of the microorganism or cell by contacting an extracting agent containing a surfactant with a solution that may contain the microorganism or cell, the solution to be tested may be filtered through a filter (this step may also be referred to as step (o)). During this step, while the filter (for example, a cellulose acetate filter) may also adsorb a very small amount of the surfactant, this is not encompassed by "adsorbing a surfactant" as used herein as described above.

In a certain embodiment, the kit for detecting a microorganism or cell of the present disclosure is a kit for detecting a microorganism or cell in a solution. In the present specification, unless otherwise specified , the filter refers to a filter for filtering a liquid. Apart from this, a member for filtering a gas shall be referred to as an air filter in the present specification. In a certain embodiment, the kit for detecting a microorganism or cell of the present disclosure does not comprise an air filter. In a certain embodiment, a kit having an air filter is excluded from the kit for detecting a microorganism or cell of the present disclosure.

### [RNase]

In a certain embodiment, the kit of the present invention may comprise RNase. Moreover, in a certain embodiment, the method of the present invention may comprise using RNase. The RNase here means an RNase not derived from the sample.

As used herein, RNase means an enzyme that catalyzes a reaction that produces 5'-mononucleotide (AMP, GMP, CMP, and UMP) from RNA, and examples thereof include the following: (1) Endonuclease S₁ (EC3.1.30.1), (2) Venom exonuclease (EC3.1.15.1), (3) Phosphodiesterase 1 (EC3.1.4.1). Endonuclease S₁ includes Nuclease P₁, Mung beans nuclease, and Neurospora crassa nuclease.

In another embodiment, the kit of the present invention does not comprise RNase or does not comprise a substantial amount of RNase. Moreover, in a certain embodiment, the method of the present invention does not comprise using RNase or does not comprise using a substantial amount of RNase. In this embodiment, RNases derived from the sample may be contained in the reaction system. As used herein, the "substantial amount of RNase" means an amount of RNase that does not affect the effect (for example, the effect of providing a method for accurate detection of contamination, that is not susceptible to ATP-degrading activity) of the kit or method of the present invention. Examples of kits or methods not comprising a substantial amount of RNase include a kit comprising RNase, for example, at a final concentration of 0.3 U/ml or less, 0.15 U/ml or less, 0.1 U/ml or less, 0.05 U/ml or less, 0.01 U/ml or less, or 0.001 U/ml or less in the reaction system, or a method comprising using such amount of RNase. In the present specification, the enzyme unit of RNase is defined as an amount of enzyme having an activity unit (U) of the enzyme having the RNA-degrading ability that converts 1.0 µmole per minute of substrate into acid-soluble nucleotide at 37°C, by focusing on the RNA-degrading ability of the enzyme. For example, the enzyme unit of Nuclease P₁ is defined as an amount of enzyme that converts 1.0 µmole of substrate per minute into acid-soluble nucleotide at pH 5.3 at 37°C (for details of the definition of the enzyme activity of Nuclease P₁, see the catalogue of Merck & Co., Inc. (http://www.sigmaaldrich.com/content/dam/sigma-aldrich/does/Sigma/General_Information/nuclease_p1.pdf)).

### Examples

The present invention is described in detail further referring to the Examples below. However, the technical scope of the present invention is not limited by the Examples in any way.

### (Example 1)

Surfactant benzalkonium chloride or n-tetradecyl-N,N-dimethyl-3-ammonio-1-propanesulfonate (manufactured by Merck Millipore) was prepared with sterile ultra pure water to a concentration of 0, 0.01, or 0.1% to be used in the following test. As the benzalkonium chloride, Benzalkonium Chloride OSVAN 10% (manufactured by Nihon Pharmaceutical Co., Ltd.) was used. After adding 0.1 mL of the surfactant of each of the concentrations to a cotton ball, the resultant cotton ball was centrifuged at 5000 rpm for 1 minute to collect a surfactant solution from the cotton ball portion. This operation was repeated 5 times, and the surfactant solutions thus collected were pooled into one solution. As the cotton ball used as a surfactant adsorbent, Cotton Ball Osaki Cottom Swab, Size No. 7 (manufactured by Osaki Medical Corp.) (material: cotton) was used.

Into LumiTube (Registered trademark) (manufactured by Kikkoman Biochemifa Company), 0.1 mL of a luminescence reagent prepared to have a composition shown in Table 1 was dispensed, 0.2 mL of the surfactant solution of each of the concentrations collected from the cotton ball was added thereto, 0.01 mL of a 1 × 10⁻⁷ M ATP solution was quickly added thereto, and immediately thereafter, the luminescence level was measured with Lumitester (Registered trademark) C-110 (manufactured by Kikkoman Biochemifa Company). For comparison, surfactant solutions of the respective concentrations not being contacted with a cotton ball were tested in a similar manner.

**[Table 1]**

| Table 1 Composition of Luminescence Reagent | |
|---|---|
| 25 mM | Tricine |
| 15 mM | Magnesium Acetate Tetrahydrate |
| 2 mM | EDTA |
| 0.37% | Sucrose |
| 0.2 mM | Dithiothreitol (DTT) |
| 0.2 % | BSA |
| 8 µg/mL | Luciferase |
| 1.5 mM | Luciferin |

As for n-tetradecyl-N,N-dimethyl-3-ammonio-1-propanesulfonate, change of the luminescence level over time in a solution containing no n-tetradecyl-N,N-dimethyl-3-ammonio-1-propanesulfonate and a solution containing 0.01% n-tetradecyl-N,N-dimethyl-3-ammonio-1-propanesulfonate was measured with or without contact with a cotton ball.

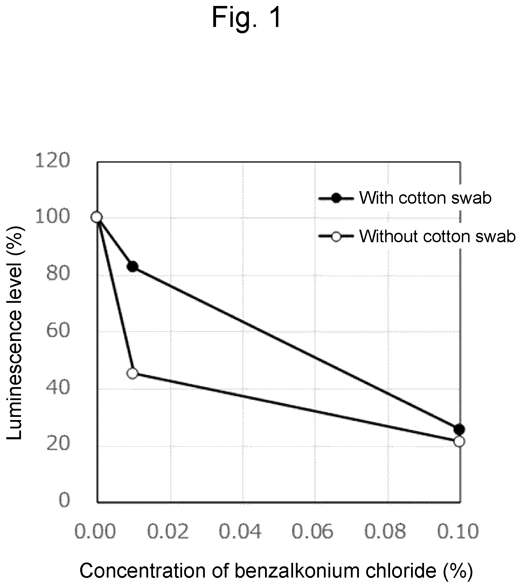

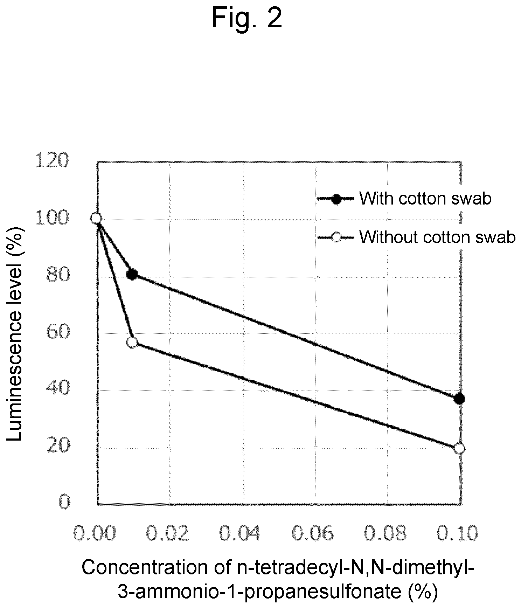

By taking the luminescence level of sterile ultra pure water containing no surfactant as 100%, the relative luminescence levels with benzalkonium chloride at the respective concentrations are illustrated in Figure 1, and the relative luminescence levels with n-tetradecyl-N,N-dimethyl-3-ammonio-1-propanesulfonates at the respective concentrations are illustrated in Figure 2.

It was revealed that in the presence of the surfactant, the luminescence levels was remarkably reduced in accordance with increasing concentration both in the case of benzalkonium chloride which is a cationic surfactant and n-tetradecyl-N,N-dimethyl-3-ammonio-1-propanesulfonate which is a zwitterionic surfactant, whereas the reduction was suppressed when the solution was contacted with a cotton ball. It is believed that this is due to the surfactant being adsorbed by the cotton ball portion, and therefore the inhibition of luciferase luminescence by the surfactant being suppressed.

Further, the change over time of the luminescence level with n-tetradecyl-N,N-dimethyl-3-ammonio-1-propanesulfonate is illustrated in Figure 3. In the solution containing no n-tetradecyl-N,N-dimethyl-3-ammonio-1-propanesulfonate (0%), the luminescence level was slightly reduced through consumption of ATP, whereas the luminescence level was greatly reduced when not contacted with the cotton ball in the solution containing 0.01% n-tetradecyl-N,N-dimethyl-3-ammonio-1-propanesulfonate. In contrast, when contacted with a cotton ball, the reduction was remarkably suppressed, and was suppressed substantially equivalently to the case where no n-tetradecyl-N,N-dimethyl-3-ammonio-1-propanesulfonate was included. The reason is believed to be as follows: When not contacted with the cotton ball, luciferase in the luminescence reagent caused luminescence while being deactivated due to the effect of n-tetradecyl-N,N-dimethyl-3-ammonio-1-propanesulfonate, whereas, in the solution contacted with a cotton ball, n-tetradecyl-N,N-dimethyl-3-ammonio-1-propanesulfonate was adsorbed by the cotton ball, and therefore, the deactivation of luciferase was suppressed.

It was revealed, based on these results, that when a solution containing a surfactant is brought into contact with a surfactant adsorbent such as cotton, the surfactant is adsorbed, and therefore is suppressed from being carried over to the luminescence reagent, resulting in an effect of suppressing inhibition of luciferase luminescence, and further suppressing enzyme deactivation.

After extracting adenine nucleotide present in a microorganism collected on a filter with an extraction reagent containing a surfactant, the resultant is not directly reacted with a luminescence reagent but instead contacted with a surfactant adsorbent such as a cotton ball, and, thereby, carry over of the surfactant to the luminescence reagent is suppressed, and luminescence is measured with effects of inhibition and deactivation being suppressed, and therefore, adenine nucleotide present in the microorganism can be measured with high sensitivity. These results verify that a cotton swab did not affect the ATP luminescence level, and yet selectively adsorbed the surfactant only, and could suppress the reduction of the luminescence level otherwise caused by the surfactant, which was highly surprising. Further, when a microorganism is first sampled by swabbing with a cotton swab, and then extraction is carried out with a surfactant and then the luminescence level is measured, the cotton swab has already been used at the stage in which the surfactant is applied, and therefore, the effect above cannot be achieved.

### (Example 2)

Benzalkonium chloride or n-tetradecyl-N,N-dimethyl-3-ammonio-1-propanesulfonate (Zwittergent (Registered trademark) 3-14, manufactured by Merck Millipore) used as a surfactant was prepared with sterile ultra pure water to a concentration of 0.01% to be used in the following test. As the benzalkonium chloride, Benzalkonium Chloride OSVAN 10% (manufactured by Nihon Pharmaceutical Co., Ltd.) was used. After adding 0.1 mL of the surfactant of each concentration to a cotton ball portion of each of the various cotton swabs described below, the resultant was centrifuged at 5000 rpm for 1 minute to collect a surfactant solution from the cotton ball portion. This operation was repeated 5 times, and the surfactant solutions thus collected were pooled into one solution. As the surfactant adsorbent, a cotton swab (material: cotton) attached to LuciPac (Registered trademark) A3 Surface (manufactured by Kikkoman Biochemifa Company, Product No.: 60361), industrial cotton swab P1502E (manufactured by J.C.B. Industry Limited) (material: cotton), industrial cotton swab S-S-P (manufactured by J.C.B. Industry Limited) (material: cotton and rayon blend), and Mentip (Registered trademark) polyester cotton swab (sterilized) 1PW1505P (manufactured by J.C.B. Industry Limited) (material: polyester) were used.

For comparison, sterile ultra pure water containing no surfactant was brought into contacted with each of the various cotton swabs and tested accordingly.

Into LumiTube (Registered trademark) (manufactured by Kikkoman Biochemifa Company), 0.1 mL of a luminescence reagent prepared to have the composition shown in Table 1 was dispensed, 0.2 mL of the surfactant of each concentration collected from the cotton swab was added thereto, 0.01 mL of a 1 × 10⁻⁷ M ATP solution was quickly added thereto, and the luminescence level was measured with Lumitester (Registered trademark) C-110 (manufactured by Kikkoman Biochemifa Company). For comparison, a surfactant solution of each concentration not brought into contact with any cotton swab was tested in a similar manner.

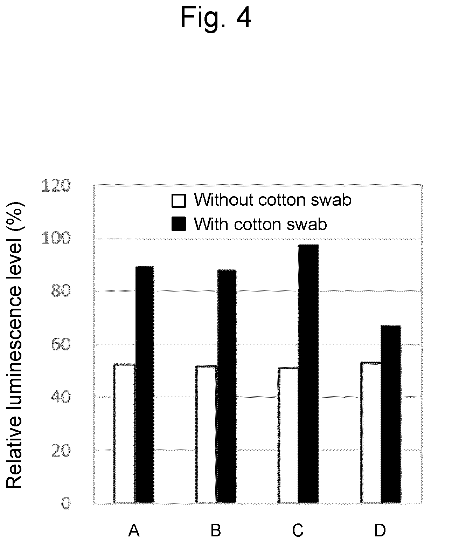

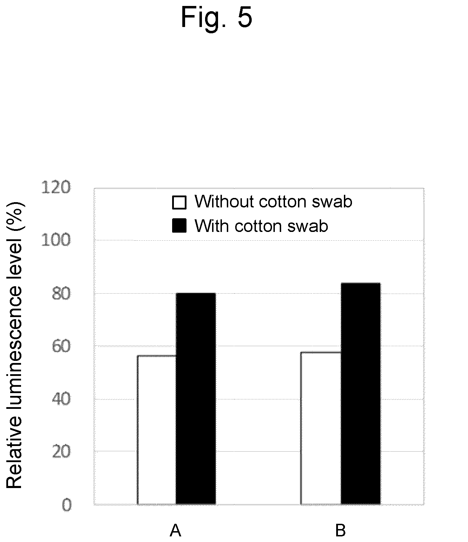

By taking the luminescence level of sterile ultra pure water containing no surfactant as 100%, the effect of suppressing inhibition of luminescence due to benzalkonium chloride achieved by various cotton swabs is illustrated in Figure 4, and the effect of suppressing inhibition of luminescence due to n-tetradecyl-N,N-dimethyl-3-ammonio-1-propanesulfonate is illustrated in Figure 5.

It was revealed that although in the presence of the surfactant, the luminescence level was remarkably reduced in accordance with the concentration, the reduction was suppressed when the surfactant was brought into contact with a cotton swab. It is believed that this is because the influence (luminescence inhibition, and deactivation) of the surfactant on luciferase is suppressed because the surfactant is adsorbed by the cotton ball portion. Moreover, it was also confirmed that this effect is obtained regardless of the type of material of the cotton ball portion and the type of surfactant. In other words, even when a cotton swab or a cotton ball portion of another material is used, or when a surfactant of another type is used for the extraction, the cotton swab or the cotton ball portion adsorbs the surfactant, and therefore, it is highly plausible that the influence on the luminescence level can be suppressed.

### (Example 3)

In order to confirm whether benzalkonium chloride, which is the main component of the extracting agent was adsorbed or not, a cationic surfactant measurement reagent "PACKTEST cationic surfactant (manufactured by KYORITSU CHEMICAL-CHECK Lab., Corp.)" was used to measure the amount of the cationic surfactant in a solution contacted with a cotton swab.

As the cotton swab, a cotton swab for LuciPac (Registered trademark) A3 Surface and an industrial cotton swab S-S-P were used. After allowing each cotton swab to absorb 0.1 mL of a 0.01% benzalkonium chloride solution, the resultant was centrifuged at 5000 rpm for 1 minute to collect a surfactant solution from the cotton ball portion. This surfactant solution was diluted 2 fold with sterile ultra pure water, 1 mL of the diluent was dispensed to be added to a tube of PACKTEST, and a reagent held in the tube was dissolved therein by pipetting.

For comparison, solutions containing benzalkonium chloride at various concentrations were diluted 2 fold to be measured in a similar manner.

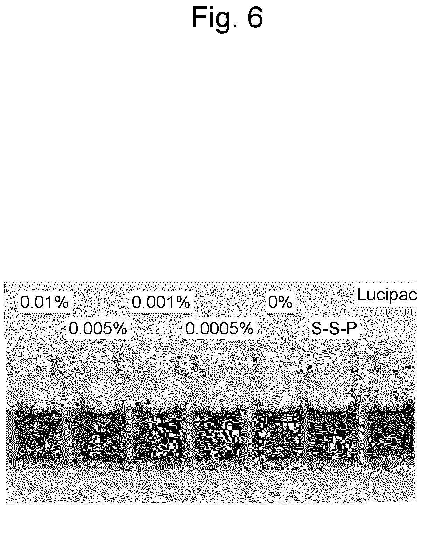

Figure 6 illustrates solutions brought into contact with the aforementioned cotton swabs and solutions containing benzalkonium chloride at various concentrations for comparison. Five solutions illustrated from the left hand side in Figure 6 are benzalkonium chloride solutions for comparison, in which blue color was exhibited at 0.01% and 0.005%, red purple was exhibited at 0%, and an intermediate color tone therebetween was exhibited at 0.0001 and 0.00005%. On the other hand, when the cotton swab for LuciPac (Registered trademark) A3 surface and the industrial cotton swab S-S-P were each used, a color tone corresponding to a benzalkonium chloride concentration between 0.005% and 0.001% was exhibited, and in other words, it could be confirmed that the concentration of surfactant that had been 0.01% before contact with the cotton swab was greatly reduced by bringing into contact with the cotton swab.

It is believed, based on these results, that when a surfactant such as benzalkonium chloride is brought into contact with a cotton swab or the like, benzalkonium chloride is adsorbed by the cotton swab, and therefore the concentration thereof is reduced. This result agrees with the results of the suppression of luminescence inhibition obtained in Example 2.

### (Example 4)

A test was performed on the following various microorganisms. More specifically, each of *Cronobacter sakazakii* IAM12660, *Klebsiella pneumoniae* NBRC14940, *Escherichia coli* ATCC25922, *Staphylococcus aureus* ATCC6538, *Pseudomonas aeruginosa* ATCC27853, *Pseudomonas fluorescence* NISL420, *Sphingomonas parapaucimobilis, Bacillus subtilis* ATCC9372, and *Bacillus cereus* NBRC3836 was inoculated in 2.5 mL of a trypto soy broth medium (manufactured by Eiken Chemical Co., Ltd.) to be cultured overnight at 30°C under shaking at 160 rpm. Each of the thus obtained culture fluids of the microorganisms was diluted 100 fold with sterile ultra pure water, and 10 mL of the resultant diluent was filtered entirely with a 10 mL syringe (manufactured by Terumo Corporation) to which a syringe filter DISMIC (Registered trademark)-25CS045AS (manufactured by Advantec Toyo Inc.) was attached.

To 10 mL of each culture diluent, 0.5 mL of Lucifer ATP Elimination Reagent (manufactured by Kikkoman Biochemifa Company, Product No.: 60254) was added, and the resultant was allowed to stand still for 30 minutes, and then filtered in a similar manner.

0.24 mL of extraction reagent (0.01% benzalkonium chloride) measured in a container in advance was aspirated through the filter having collected the microorganism, and then discharged to the container, and thus, adenine nucleotides (ATP, AMP, ADP) were extracted from the microorganism collected on the filter. For the adenine nucleotides contained in the collected extract, LuciPac (Registered trademark) II (manufactured by Kikkoman Biochemifa Company, Product No.: 60375) was used for ATP measurement, LuciPac (Registered trademark) Pen (manufactured by Kikkoman Biochemifa Company, Product No.: 60331) was used for ATP+AMP measurement, and LuciPac (Registered trademark) A3 Surface (manufactured by Kikkoman Biochemifa Company, Product No.: 60361) was used for ATP+ADP+AMP measurement. After adding 0.1 mL of each test reagent to the cotton ball portion, Lumitester (Registered trademark) C-110 (manufactured by Kikkoman Biochemifa Company) was used for the ATP measurement, and Lumitester (Registered trademark) Smart (manufactured by Kikkoman Biochemifa Company) was used for the ATP+AMP measurement and the ATP+ADP+AMP measurement for measuring luminescence levels. In the measurement of the luminescence level, in order to correct the lot-to-lot difference of various test kits, an ATP solution of a known concentration was added to each lot to measure the luminescence level. A measured value of the luminescence level obtained when 0.01 mL of the ATP solution (1 × 10⁻⁷ M) was added was defined as the luminescence level at ATP addition, a luminescence level obtained in a sample was divided by the luminescence level at ATP addition, and the resultant was multiplied by an average luminescence level of each adenine nucleotide test reagent, 3000 RLU, to obtain a corrected value (corrected value = luminescence level of analyte ÷ luminescence level at ATP addition × 3000). Moreover, the amount of ATP+ADP was calculated by subtracting the amount of ATP+AMP from the amount of ATP+ADP+ADP, and adding the resultant to the amount of ATP. (amount of ATP+ADP) = (amount of ATP+ADP+AMP) - (amount of ATP+AMP) + amount of ATP

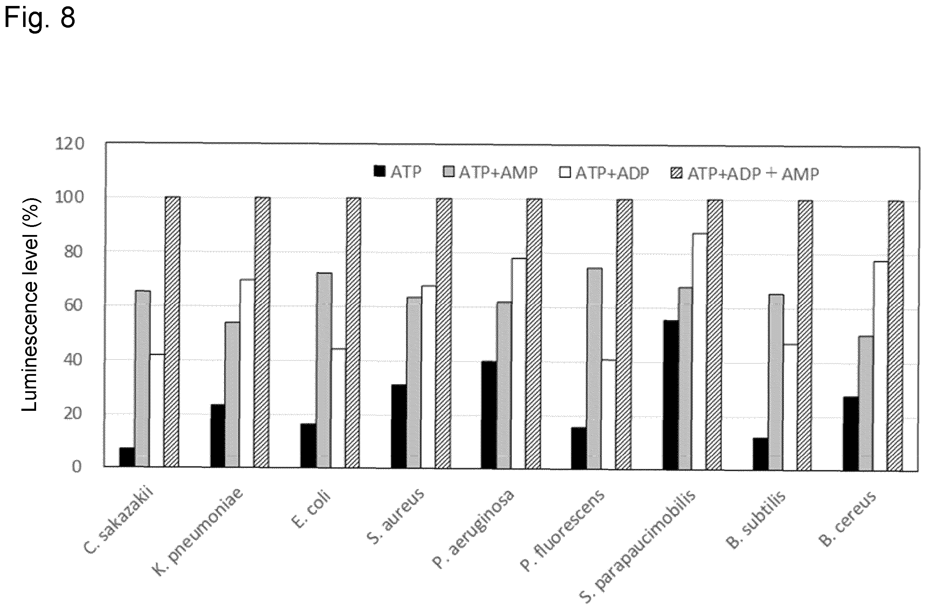

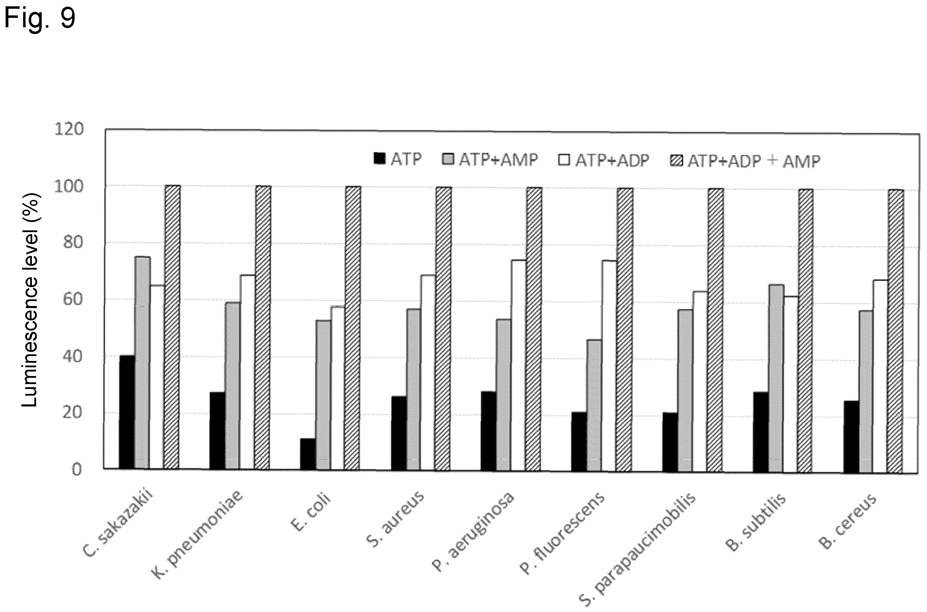

Figure 7 illustrates the outline of the present example. By taking the luminescence level (corrected value) obtained when using the ATP+ADP+AMP measurement reagent as 100%, relative luminescence levels of the respective microorganisms are illustrated in Figure 8, and relative luminescence levels obtained in using the ATP elimination reagent are illustrated in Figure 9. In all the microorganisms used in the present example, the ratios of the amount of ATP contained in the aquatic microorganisms were very low, and the ratios of the amount of ATP+AMP were also low. These results are unexpected and surprising values, upon considering that ATP+AMP is present in a ratio of 79.8 to 89.0% in a solution containing no sugar in Non Patent Literature 1. It is presumed that the different is caused because the measurement is performed in the microorganism collected with the filter in the present example which is different from Non Patent Literature 1 in which the measurement is performed in a solution. However, it was revealed that higher luminescence levels can be obtained, and highly sensitive measurement can be performed with the ATP+AMP measurement and the ATP+ADP measurement, and further with the ATP+ADP+AMP measurement as compared with the ATP measurement. It was therefore indicated that when a microorganism such as a dangerous bacterium contained in a liquid is to be detected through collection with a filter, the microorganism can be detected with high sensitivity by measuring not only ATP but also measuring ATP+AMP and measuring ATP+ADP, and further measuring ATP+ADP+AMP.

### (Example 5)

Each of *Klebsiella pneumoniae* NBRC14940, *Saccharomyces cerevisiae* NISL3399, *Pseudomonas aeruginosa* ATCC27853, *Micrococcus luteus* IFO3333, *Bacillus cereus* NBRC3836, *Cronobacter sakazakii* IAM12660, *Escherichia coli* ATCC25922, and *Staphylococcus aureus* ATCC6538 was inoculated in 2.5 mL of a trypto soy broth medium (manufactured by Eiken Chemical Co., Ltd.) and cultured overnight at 30°C under shaking at 160 rpm. Each of the thus obtained culture fluids of the microorganisms was diluted with sterile ultra pure water to prepare microorganism diluents diluted every 10 fold. 10 mL of the culture diluent obtained by diluting each microorganism culture fluid 100 fold with sterile ultra pure water was filtered entirely, to collect the microorganism, with a 10 mL syringe (manufactured by Terumo Corporation) to which each of various syringe filters was attached. As the filters, among those shown in Table 2, filters manufactured by Advantec Toyo Inc., GVS Filter Technology, and Merck Millipore were used. All the filters used had a pore size of 0.45 µm. 0.24 mL of extraction reagent (0.01% benzalkonium chloride) measured in a container was aspirated through the filter having collected the microorganism, and then discharged to the container, and thereby, adenine nucleotides (ATP, AMP) were extracted from the microorganism collected on the filter. The resultant adenine nucleotide extract was sampled with a cotton swab of LuciPac (Registered trademark) Pen (manufactured by Kikkoman Biochemifa Company, Product No.: 60331), and the luminescence level was measured with Lumitester (Registered trademark) Smart (manufactured by Kikkoman Biochemifa Company). Moreover, 0.1 mL of each microorganism suspension appropriately diluted was applied on R2A medium (manufactured by Nissui Pharmaceutical Co., Ltd.) to be cultured at 30°C overnight, and a colony thus formed was measured to obtain a viable cell count.

Likewise, *E. coli* was used for measuring various amounts of ATP+ADP+AMP with LuciPac (Registered trademark) A3 Surface (manufactured by Kikkoman Biochemifa Company, Product No.: 60361). As filters, filters manufactured by Advantec Toyo Inc., GVS Filter Technology, Membrane Solutions, and Merck Millipore shown in Table 2 were used.

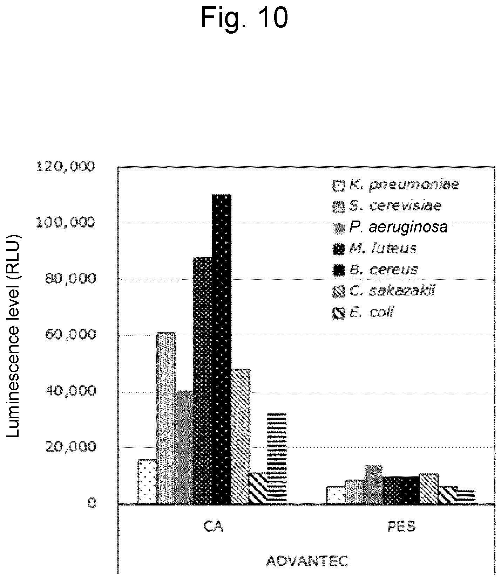

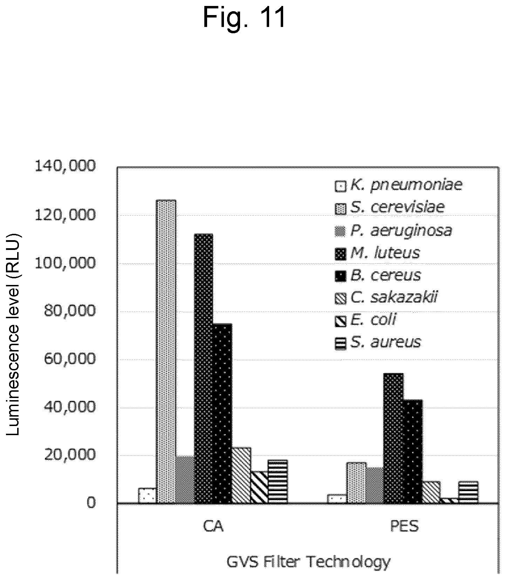

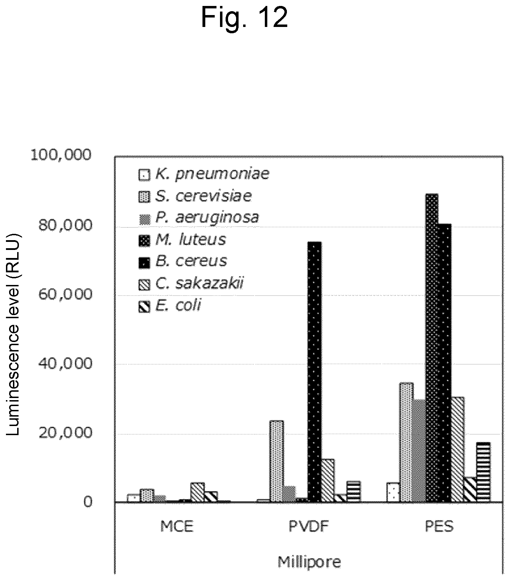

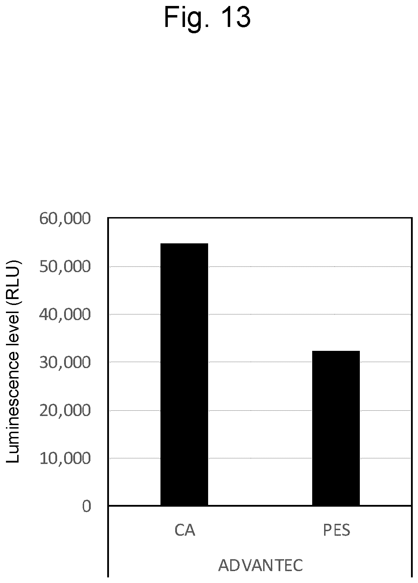

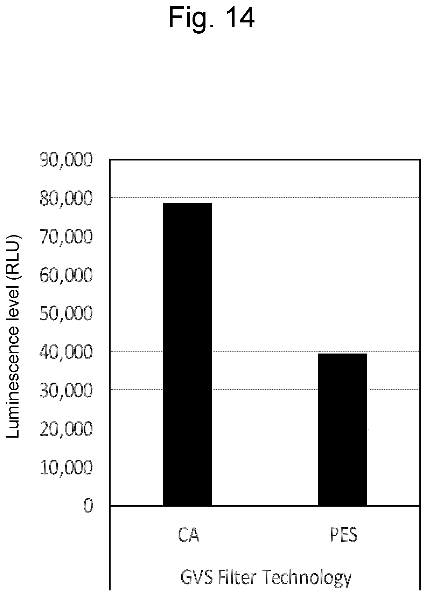

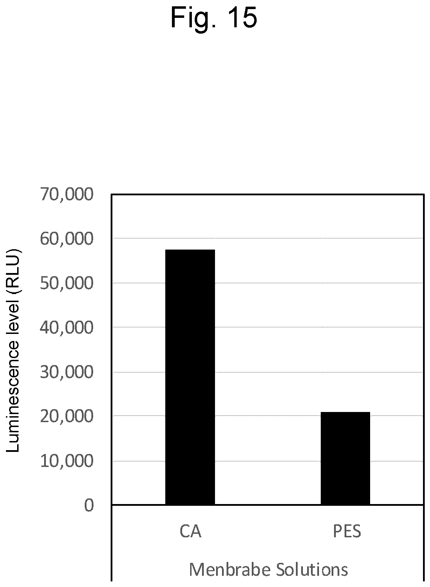

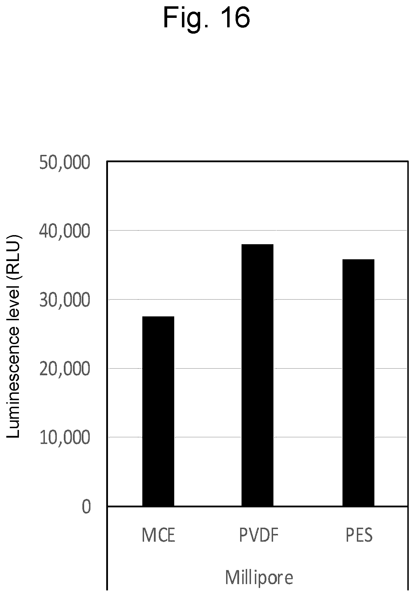

Since the housing structure and the like of the filter unit is different among the manufacturers and this may affect extraction efficiency, the luminescence levels of the microorganisms were compared for a difference depending on the material of the filters of the same manufacturer. The luminescence levels obtained in the ATP+AMP measurement of the microorganisms using the filters of Advantec Toyo Inc. are illustrated in Figure 10, the luminescence levels likewise obtained using the filters of GVS are illustrated in Figure 11, and the luminescence levels obtained using the filters of Merck Millipore are illustrated in Figure 12. Likewise, the luminescence levels obtained in the ATP+ADP+AMP measurement of *E. coli* using the filters of Advantec Toyo Inc. are illustrated in Figure 13, the luminescence level similarly obtained using the filters of GVS are illustrated in Figure 14, the luminescence levels obtained using the filters of Membrane solutions are illustrated in Figure 15, and the luminescence levels obtained using the filters of Merck Millipore are illustrated in Figure 16.

Upon using the filters of Advantec Toyo Inc., GVS, and Membrane Solutions, it is understood, by comparing cellulose acetate (CA) and polyether sulfone (PES), that the luminescence level was higher using CA, and that CA could extract a larger amount of adenine nucleotides from the microorganisms. The following is understood in comparison among the filters of Merck Millipore. The luminescence level obtained when using PES was the highest in the ATP+AMP measurement of the microorganisms, larger amounts of the microorganisms could be extracted than using polyvinylidene fluoride (PVDF) and cellulose mixed ester (MCE), and the luminescence levels obtained using PES were substantially equivalent to those obtained using PVDF and higher than those obtained using MCE in the ATP+ADP+AMP measurement. Upon considering all of these results, CA can extract largest amounts of adenine nucleotides (ATP, ADP, and AMP) from microorganisms collected on a film. Since cellulose mixed ester (MCE) also contains cellulose acetate (CA) as a component, it was predicted based on common general knowledge that there would be no or substantially no difference in performance between these materials. However, it was confirmed through the experiments that the luminescence level obtained by a cellulose acetate (CA) filter is actually higher than that obtained by a cellulose mixed ester (MCE) filter. This result was highly surprising. It is believed that similar results would be obtained when a cell is used as the analyte.

**[Table 2]**

| Table 2 Syringe Filter Used | | |
|---|---|---|
| Manufacturer | Product Name | Material of Film |
| Advantec Toyo Inc. | DISMIC (Registered trademark)-25AS | Cellulose Acetate (CA) |
| | DISMIC (Registered trademark)-28SP | Polyether Sulfone (PES) |
| GVS Filter Technology | Syringe Filter CA | Cellulose Acetate (CA) |
| | Syringe Filter PES | Polyether Sulfone (PES) |
| Membrane Solutions | MS PES Syringe Filter | Polyether Sulfone (PES) |
| | MS CA Syringe Filter | Cellulose Acetate (CA) |
| Merck Millipore | Millex (Registered trademark)-HA | Cellulose Mixed Ester (MCE) |
| | Millex (Registered trademark)-HV | Polyvinylidene Fluoride (PVDF) |
| | Millex (Registered trademark)-HP | Polyether Sulfone (PES) |

### Industrial Applicability

According to the present invention, a microorganism or cell can be detected by measuring an adenine nucleotide.

All publications, patents, and patent applications cited herein shall be incorporated herein by reference as they are.

## Claims

1. A method for detecting a microorganism or cell, comprising the steps of:
(i) extracting an adenine nucleotide component of the microorganism or cell by bringing an extracting agent containing a surfactant into contact with a solution that may contain the microorganism or cell without allowing any surfactant adsorbent to be in contact with the solution that may contain the microorganism or cell;
(ii) bringing an analyte comprising the adenine nucleotide component derived from the microorganism or cell, resulting from the extraction of step (i), into contact with a surfactant adsorbent;
(iii) bringing the sample contacted with the surfactant adsorbent into contact with a luminescence reagent comprising luciferase and luciferin, or suspending the sample contacted with the surfactant adsorbent in a solution and then bringing the solution suspending the sample into contact with a luminescence reagent comprising luciferase and luciferin; and
(iv) measuring luminescence level.

2. The method according to claim 1, wherein the surfactant adsorbent comprises cotton, rayon, cellulose, or polyester.

3. The method according to claim 2, wherein the extracting agent comprises one or more of a surfactant, a cationic surfactant, an anionic surfactant, an amphoteric surfactant, a nonionic surfactant, or benzalkonium chloride and n-tetradecyl-N,N-dimethyl-3-ammonio-1-propanesulfonate.

4. The method according to claim 3,
wherein the method is a method for detecting a microorganism or cell,
the extracting agent is an extracting agent for a microorganism or an extracting agent for a cell for extracting ATP from the microorganism or cell,
the adenine nucleotide is ATP, and
step (iii) includes allowing ATP to cause luminescence by luciferase.

5. The method according to claim 4, wherein the luminescence reagent further comprises an enzyme that produces ATP from AMP.

6. The method according to claim 4, wherein the luminescence reagent further comprises an enzyme that produces ATP from ADP.

7. The method according to claim 4, wherein the luminescence reagent further comprises an enzyme that produces ATP from AMP, and an enzyme that produces ATP from ADP.

8. The method according to claim 4, wherein the luminescence reagent further comprises an enzyme that produces ADP from AMP, an enzyme that produces ADP from ATP and AMP, and an enzyme that produces ATP from ADP.

9. The method according to claim 4, wherein the luminescence reagent further comprises an enzyme that produces ATP from AMP, and an enzyme that produces AMP from ADP.

10. The method according to claim 1, further comprising, prior to step (i), step (o) of filtering the solution to be tested through a filter to provide the solution that may contain the microorganism or cell.

11. The method according to claim 10, wherein the filter is a cellulose acetate filter.

12. A kit for detecting a microorganism or cell, comprising:
(i) an extracting agent comprising a surfactant;
(ii) a sampling portion comprising a surfactant adsorbent;
(iii) a luminescence reagent comprising luciferase and luciferin; and
(iv) instructions for use,
wherein the instructions for use states detecting the microorganism or cell by the steps of:
(A) extracting an adenine nucleotide component of the microorganism or cell by bringing the extracting agent comprising the surfactant into contact with a solution that may contain the microorganism or cell without bringing any surfactant adsorbent into contact with the solution that may contain the microorganism or cell;
(B) collecting an analyte comprising the adenine nucleotide component derived from the microorganism or cell, resulting from the extracting of step (A), by using the sampling portion comprising a surfactant adsorbent;
(C) bringing the sample collected with the sampling portion into contact with the luminescence reagent comprising luciferase and luciferin; and
(D) measuring luminescence level.

13. The kit according to claim 12, wherein the surfactant adsorbent comprises cotton, rayon, cellulose, or polyester.

14. The kit according to claim 13, wherein the extracting agent comprises one or more of a surfactant, a cationic surfactant, an anionic surfactant, an amphoteric surfactant, a nonionic surfactant, benzalkonium chloride and n-tetradecyl-N,N-dimethyl-3-ammonio-1-propanesulfonate.

15. The kit according to claim 12,
wherein the kit is a kit for detecting a microorganism or cell,
the extracting agent is an extracting agent for a microorganism or an extracting agent for a cell for extracting ATP from the microorganism or cell,
the adenine nucleotide is ATP, and
step (C) includes allowing ATP to cause luminescence by luciferase.

16. The kit according to claim 15, wherein the luminescence reagent further comprises an enzyme that produces ATP from AMP.

17. The kit according to claim 15, wherein the luminescence reagent further comprises an enzyme that produces ATP from ADP.

18. The kit according to claim 15, wherein the luminescence reagent further comprises an enzyme that produces ATP from AMP, and an enzyme that produces ATP from ADP.

19. The kit according to claim 15, wherein the luminescence reagent further comprises an enzyme that produces ADP from AMP, an enzyme that produces ADP from ATP and AMP, and an enzyme that produces ATP from ADP.

20. The kit according to claim 15, wherein the luminescence reagent further comprises an enzyme that produces ATP from AMP, and an enzyme that produces AMP from ADP.

21. The kit according to claim 12, further comprising a filter.

22. The kit according to claim 21, wherein the filter is a cellulose acetate filter.
